Europäisches Patentamt
European Patent Office
Office européen des brevets

Publication number: 0 367 723 A2

# EUROPEAN PATENT APPLICATION

Application number: 89810807.1

Int. Cl.5: A61L 2/00 , A61L 2/22

Date of filing: 26.10.89

Priority: 04.11.88 US 267025

Date of publication of application: 09.05.90 Bulletin 90/19

Designated Contracting States: AT BE CH DE ES FR GB GR IT LI LU NL SE

Applicant: CIBA-GEIGY AG
Klybeckstrasse 141
CH-4002 Basel(CH)

Inventor: Schweigl, Erwin
87 Garside Crescent
Bramalea Ontario L6S 1H5(CA)
Inventor: Hayes, Thomas Peter
4 Cullum Drive
Carlisle Ontario L0R 1H0(CA)
Inventor: Kljajic, Dusan
48 Allanhurst Drive
Islington, Ontario M9A 4K1(CA)
Inventor: Jellositz, Rolf
1144 Kos Boulevard
Mississauga Ontario L5J 4L6(CA)

## Method for producing a sterile preservative-free aerosol solution.

Described is a method, and the resulting product, for producing a sterile preservative-free aerosol product, e.g. a contact lens solution, comprising: aseptically filling a pre-sterilized aerosol container with a solution to be dispensed preserved with sufficient hydrogen peroxide; aseptically introducing into the container means, such as a catalyst, for converting the hydrogen peroxide into inert or inactive substances one of which is a suitable propellant; and sealing and storing the container for a time sufficient to allow complete inactivation of the hydrogen peroxide and creation of sufficient propellant.

EP 0 367 723 A2

Xerox Copy Centre

# METHOD FOR PRODUCING A STERILE PRESERVATIVE-FREE AEROSOL SOLUTION

This invention relates to a method for producing a sterile, preservative-free, aerosol solution product. e.g. for use by contact lens wearers, such as a sterile, preservative-free, aerosol saline solution product and more particularly to a method which utilizes a preservative together with means for converting the preservative into an inert substance which becomes the propellant of the aerosol product.

Wearers of contact lenses must regularly rinse their lenses with a saline solution prior to inserting the lenses for continued use. For example, contact lenses must be rinsed after subjecting the lenses to a variety of chemical disinfection regimens necessary to rid the lenses of pathogenic microorganism. The saline solution typically has the same tonicity as human tears (isotonicity) to minimize ocular irritation in the user. Rinsing the lenses with a normal saline solution will wash away potentially harmful and irritating chemicals remaining on the lenses as a result of a disinfection regimen.

Contact lens users generally react unfavourably, by experiencing untoward reactions, to the presence of a variety of preservatives commonly used in contact lens solutions, including normal saline solutions. It is desirable therefore to be able to produce contact lens products, including normal saline solutions, which are sterile, yet free of preservatives. Such solutions must be sterile in order not to introduce harmful micro-organisms into the eyes of the contact lens user.

It is known in the prior art (see U.S. Patent No. 3,912,451) to place a saline solution preserved with hydrogen peroxide into a container together with a disk coated with platinum. The platinum reacts as a catalyst with the hydrogen peroxide to cause this preservative to become inert by splitting it into water ($H_2O$) and oxygen ($O_2$) gas. The complete reaction is as follows:

$2H_2O_2$ + Platinum Catalyst $\rightarrow 2H_2O + O_2$

The prior art method utilizing the above rection involves the contact lens user adding the solution preserved with hydrogen peroxide to a non-sterile container, placing the platinum coated disk into the container, adding the contact lenses to the container and leaving the lenses to soak over night, so that by the next morning, the hydrogen peroxide will have been converted into inert water and the lenses will be soaking in pure saline solution. The contact lens user would then merely insert the lenses without risk of irritation from chemical preservatives. This prior art method is however, cumbersome, awkward and time consuming. A further prior art method (see U.S. Patent 4,585,488) uses catalase in a two-step, but otherwise similar method.

It is also known in the prior art to modify the aforesaid method by utilizing sodium pyruvate to decompose the hydrogen peroxide (see U.S. Patent No. 4,521,375). The sodium pyruvate recacts with the hydrogen peroxide to produce inert by-products including water and carbon dioxide.

It is further known in the prior art to fill an aerosol can with normal saline solution and a propellant, and then to subject the sealed aerosol can to terminal sterilization by irradiating it with gamma rays. This results in a sterile aerosol product containing normal saline solution, and free of any chemical preservatives. Such prior art aerosol products require the addition of a propellant, such as nitrogen, in order to achieve the pressure levels necessary for proper aerosol functioning. The disadvantages of such prior art irradiated aerosol products are several. Firstly, it is very expensive to conduct terminal sterilization by irradiation using gamma rays. Secondly, there are safety and hence political concerns relating to the use of radiation to sterilize products for use in the human body. Lastly, there are several practical problems related to the use of radiation, including the need to irradiate the aerosol product very shortly after the aerosol container has been filled and the fact that use of radiation on some chemicals will cause the chemicals to breakdown. This limits which chemicals may be used in such products to be sterilized by radiation.

An additional problem present in the prior art aerosol products relates to the difficulty in obtaining United States Federal Drug Administration approval for aseptic packaging of unpreserved solutions for ophthalmic use or equivalent approval in another country.

It is desirable to have sterile, preservative-free ophthalmic products in aerosol dispensing containers in order to maintain product sterility during use. Because the aerosol container is under pressure, there is minimal risk of micro-organisms being able to enter the sterile environment inside the aerosol can during its use. Ophthalmic solutions housed in plastic squeeze bottles may easily become contaminated during use, due to reflux action of the liquid during dispensing of the solution by the contact lens user. As a result of the aforesaid disadvantages, there is a need for an economical, safe and effective ophthalmic saline solution in aerosol packaging, which is free of chemical preservatives at the time of use of the solution by the contact lens wearer, without having been subjected to radiation exposure.

Presently the only unpreserved contact lens solutions marketed are either blow fill sealed or are subjected to terminal sterilization by irradiation. The blow fill sealed technique is currently used for

aseptically packaged unpreserved solutions since the process involves no human intervention. When using terminal sterilization, the product is not aseptically filled but rather the packaged product is subjected to sterilization by irradiation with gamma rays which will destroy all microbiological activity.

In a conventional bottle filling operation, human intervention in a clean room prevents FDA approval of packaging aseptically unpreserved solutions.

The method of the instant invention has overcome the prior art problems by utilizing the method of the invention as described herein.

In its broadest aspect, this invention teaches combining a preservative and a product to be dispensed in an aerosol container with means for converting the preservative into an inert or inactive substance, which substance is suitable to act as the propellant of the aerosol product to be dispensed.

The instant invention contemplates not only the method described herein, but in addition contemplates the end product, namely a preservative free aerosol product when prepared by the method described.

Preferably, the preservative is hydrogen peroxide. It is further preferred that the container is a one-chamber container wherein the fluid to be dispensed is in intimate contact with, e.g. mixed with the preservative, being preferably hydrogen peroxide, such that even the propellant, being one of the reaction products of chemical reaction of the preservative, preferably hydrogen peroxide, with the means for converting the preservative into an inert or inactive substance, suitable to act as a propellant, is in intimate contact with the fluid to be dispensed.

Preferably this invention provides a method for producing a sterile preservative-free aerosol product comprising:

(a) aseptically filling a presterilized aerosol container with a solution to be dispensed preserved with sufficient hydrogen peroxide;

(b) aseptically introducing into the aerosol container means for converting the hydrogen peroxide into inert or inactive substances one of which is a propellant;

(c) sealing and

(d) storing the aerosol container for a time sufficient to allow complete inactivation of the hydrogen peroxide and creation of sufficient propellant to dispense the solution from said aerosol container, wherein the sequence of steps (b) and (c) may be reversed where appropriate.

Preferably, the aerosol product to be dispensed is a saline solution, e.g. a saline solution having 0.7 to 1.1 % sodium chloride, preferably 0.8 to 1.0 % sodium chloride, most preferably an isotonic sodium chloride solution ("normal saline").

More particularly, this invention provides a method for producing a sterile preservative-free aerosol saline solution comprising: a) aseptically filling a presterilized aerosol container with a saline solution to be dispensed preserved with sufficient hydrogen peroxide; b) aseptically introducing into the aerosol container means for converting the hydrogen peroxide into inert or inactive substances one of which is a suitable propellant; c) sealing and d) storing the aerosol container for a time sufficient to allow complete inactivation of the hydrogen peroxide and creation of sufficient propellant to dispense the saline solution from said aerosol container, wherein the sequence of steps b) and c) may be reversed where appropriate, e.g. when using catalase as the means for decomposing hydrogen peroxide. Generally it is preferred to seal the container following step b). However, if the propellant, by reaction of hydrogen peroxide with the means for converting it, is created almost immediately after contacting both compounds, the reverse procedure is appropriate and therefore recommended.

The invention is particularly applicable to making products for use by contact lens users including saline solutions. Furthermore, it should be noted that this invention has application beyond the preparation of contact lens solutions. For example, the invention is further applicable to the preparation of aseptic medicinal burn (or other medicinal) sprays and aerosol food products (where the food product is compatible with hydrogen peroxide). Still further the invention is applicable to the preparation of preservative-free cosmetic products in aerosol or atomizer type containers.

The means utilized for converting the hydrogen peroxide into inert by-products may be several. It is highly recommended to use either a catalyst and/or a reducing agent. It is apparent to those skilled in the art which substances are suitable hydrogen peroxide converting or decomposing catalysts or reducing agents. Preferred is the use of platinum which acts as a catalyst to convert the hydrogen peroxide into water and oxygen gas. Also preferred is the use of catalase enzyme as the means for converting the hydrogen peroxide to inert substances. Sufficient hydrogen peroxide must be used in order to provide acceptable preservation of the solution and furthermore to produce sufficient oxygen gas by-product to act as the propellant of the solution in the aerosol product. A general guideline together with a specific example for calculation of what may be understood by sufficient hydrogen peroxide and sufficient oxygen gas is found in example 3.

Other means for converting the hydrogen peroxide to inert substances include the use of pyruvic acid or a salt thereof, e.g. an alkali metal salt, such as sodium pyruvate. When sodium pyruvate is used, the resulting propellant is carbon dioxide gas.

In the most preferred embodiment the invention utilizes hydrogen peroxide and platinum.

The hydrogen peroxide is aseptically added to the normal saline or other type of solution in a pre-sterilized aerosol type container which is often a can. The platinum catalyst may be utilized in a variety of forms including a disk coated with platinum and commercially available and known as the AO Disc (registered trade mark of CIBA Vision Corporation). Sufficient hydrogen peroxide must be added to obtain the desired preservation characteristics. Furthermore, sufficient hydrogen peroxide must be present to provide enough oxygen (or other suitable) gas to act as propellant of the ultimate aerosol product. Sufficient propellant must be present to expel, under pressure, substantially the entire contents of the aerosol or other type of spray product. The platinum catalyst when employed as a platinum coated disc may be inserted into the aerosol container where it is free to move about the contents thereof when the container is agitated. As a catalyst, the platinum is utilized in the chemical reaction without contaminating the solution being the end product of the reaction resulting from the inactivation of the hydrogen peroxide.

Once all ingredient components have been added to the container, it is aseptically sealed and stored for a time sufficient to allow for both the inactivation of the hydrogen peroxide to be completed and the creation of sufficient propellant, all prior to use of the product by the contact lens wearer (as the case may be).

It is within the disclosure of the present invention that such aerosol products need not be restricted to substances conventionally regarded as "solutions", but rather may include foams, a variety of emulsions or any other liquid or semi-liquid composition compatible with an aerosol or other spray type of product.

Furthermore, it should be noted that this invention has application beyond the preparation of contact lens solutions. For example, burn products are preferred in aerosol form, in order to provide uniform and painless application to the burn area. Using the methods of the instant invention, it is possible to provide a sterile, preservative-free aerosol burn spray as follows:

$$\text{Medicament} + 2H_2O_2 + \underset{\text{Catalyst}}{\text{Platinum}} \rightarrow 2H_2O + O_2 + \text{Medicament}$$

The method of the instant invention has further application to food products. Utilizing the invention it is possible to incorporate food products compatible with hydrogen peroxide into an aerosol or other spray type container. After the hydrogen peroxide has been inactivated by a catalyst or reducing agent, the food product may be sprayed from the container and incorporated into other foods or directly consumed by humans, for example, in accordance with the following chemical reaction:

$$2H_2O_2 + \text{catalyst or reducing agent} + \text{compatible food} \rightarrow 2H_2O + O_2 + \text{food}$$

Many users of cosmetic products suffer allergic (dermatological reactions) to ingredients of such products including preservatives. Using the methods of the instant invention, it is possible to provide a sterile, preservative-free cosmetic spray product. An example of such a reaction is as follows:

$$2H_2O_2 + \text{catalyst} + \text{compatible cosmetic} \rightarrow 2H_2O + O_2 + \text{cosmetic}$$

It will also be within the scope of this invention that the method according to the invention herein may also work with preservatives other than hydrogen peroxide, as long as such preservatives are capable of reaction to inert substances one of which is a propellant.

By way of examples set out hereinafter, there are variations of the method of the invention as well as calculations employed in determining quantitites of preservative to be used.

<u>Example 1:</u> When using catalase enzyme to inactivate hydrogen peroxide, the following chemical

reaction takes place:

$2H_2O_2$ + Catalase Enzyme → $2H_2O + O_2$

The catalase enzyme promotes the decomposition of the hydrogen peroxide to water and oxygen gas. Catalase enzymes are available from a variety of sources such as animals, plants, bacteria and fungi. Because catalase reacts immediately with hydrogen peroxide, when using catalase the following procedure is recommended. The can should be capped after being filled with hydrogen peroxide and normal saline (or other substance). Thereafter the catalase may be injected through the valve into the can preferably using aseptic technique in a clean room.

Example 2: When sodium pyruvate is employed in the performance of the method of the invention, the following chemical reaction occurs:

$H_2O_2 + CH_3COCO_2Na \rightarrow CH_3CO_2Na + CO_2 + H_2O$

It will be apparent from the above chemical reaction, that the use of sodium pyruvate to inactivate the hydrogen peroxide results in the propellant being carbon dioxide gas instead of oxygen.

Example 3: The following calculations illustrate the quantity of hydrogen peroxide required to pressurize an aerosol can containing saline solution.

- assume that the can has an internal volume of 560 ml,
- if the can is filled with 360 ml of solution, the resulting headspace will be 200 ml,
- assume that the oxygen produced by the decomposition of peroxide behaves like an ideal gas,
- therefore the ideal gas law (pV = nRT) applies,

where: p = pressure (atmospheres)

V = volume ($cm^3$)

n = amount of gas (gram moles)

T = temperature (Kelvin)

$$R = \text{gas constant} \quad 82.05 \left[\frac{cm^3 \cdot atm}{g.mole \cdot K}\right],$$

- assuming the following conditions:

V = 200 ml, p = 90 psig (6.12 atm), T = 20° C (293 K), by the ideal gas law, n = 0.509 g.moles of oxygen,

- based on the stochiometry of the reaction, the required amount of hydrogen peroxide is 0.102 g.moles; this converts to 3.46 g of oxygen,
- typically, the hydrogen peroxide is in a 35 % w/v solution,
- therefore the required volume of solution is 9.9 ml.

This calculation shows that in order to pressurize a 560 ml can, filled with 360 ml solution, to an internal pressure of 90 psig at 20° C, 9.9 ml of 35 % (w/v) peroxide solution should be added to the saline solution. Experiments show that these calculations are an accurate representation of the actual reaction.

## Claims

1. A method for producing a sterile preservative-free aerosol product comprising:

(a) aseptically filling a presterilized aerosol container with a solution to be dispensed preserved with sufficient hydrogen peroxide;

(b) aseptically introducing into the aerosol container means for converting the hydrogen peroxide into inert or inactive substances one of which is a propellant;

(c) sealing and

(d) storing the aerosol container for a time sufficient to allow complete inactivation of the hydrogen peroxide and creation of sufficient propellant to dispense the solution from said aerosol container, wherein the sequence of steps (b) and (c) may be reversed where appropriate.

2. The method of claim 1 wherein the means for converting the hydrogen peroxide into inert or inactive substances is a catalyst or a reducing agent.

3. The method of claim 2 wherein the catalyst is platinum.

4. The method of claim 2 wherein the catalyst is catalase, and the container is sealed prior to the introduction of the catalase into the container.

5. The method of claim 4 wherein the container is an aerosol can with a valve and the catalase is introduced into the sealed can by injecting it into the can through the valve.

6. The method of claim 2 wherein the reducing agent is sodium pyruvate.

7. A method for producing a sterile preservative-free aerosol saline solution comprising: a) aseptically filling a presterilized aerosol container with a saline solution to be dispensed preserved with sufficient hydrogen peroxide: b) aseptically introducing into the aerosol container means for converting the hydrogen peroxide into inert or inactive substances one of which is a suitable propellant; c) sealing and d) storing the aerosol container for a time sufficient to allow complete inactivation of the hydrogen peroxide and creation of sufficient propellant to dispense the saline solution from said aerosol container, wherein the sequence of steps b) and c) may be reversed where appropriate.

8. The method of claim 7 wherein the means for converting the hydrogen peroxide into inert or inactive substances is a catalyst or a reducing agent.

9. The method of claim 7 wherein a 0.8 to 1.0 % sodium chloride solution is used.

10. The method of claim 8 wherein the catalyst is platinum.

11. The method of claim 8 wherein the catalyst is catalase and the container is sealed prior to the introduction of the catalase into the container.

12. The method of claim 11 wherein the container is an aerosol can with a valve and the catalase is introduced into the sealed can by injecting it into the can through the valve.

13. The method of claim 8 wherein the reducing agent is sodium pyruvate.

14. The method of claim 8 wherein the aerosol product contains normal saline, and the catalyst is platinum.

15. The method of claims 3, 4 or 6 wherein the aerosol product contains a medicinal spray, a food or a cosmetic.

16. A sterile preservative-free aerosol product produced by the method of any one of claims 1-6.

17. A sterile preservative-free aerosol product produced by the method of any one of claims 7-14.

18. A sterile preservative-free aerosol product produced by the method of claim 15.